# FASCICULE DE BREVET EUROPEEN

(11) **EP 1 341 505 B1**
(45) Date de publication et mention de la délivrance du brevet: **28.02.2007**
(21) Numéro de dépôt: 01999220.5
(22) Date de dépôt: 22.11.2001
(51) Int. Cl.: A61Q 5/10, A61K 8/34, A61K 8/39, A61K 8/86

(54) **COMPOSITION DESTINEE A LA TEINTURE D'OXYDATION DES FIBRES KERATINIQUES COMPRENANT DE LA GLYCERINE ET UN POLYOL DIFFERENT DE LA GLYCERINE DANS UN RAPPORT DONNE**
OXIDATIONSFÄRBEZUSAMMENSETZUNG FÜR KERATINÖSE FASERN MIT GLYCERIN UND EINEM ANDEREN POLYOL ALS GLYCERIN IN VORGEGEBENEM VERHÄLTNIS
OXIDATION DYEING COMPOSITION FOR KERATINOUS FIBRES COMPRISING GLYCERINE AND A POLYOL OTHER THAN GLYCERINE IN A SPECIFIC RATIO

(30) Priorité: 04.12.2000 FR 0015683
(43) Date de publication de la demande: 10.09.2003
(73) Titulaire: L'ORÉAL, 75008 Paris (FR)
(72) Inventeur: COTTARD, François, F-92400 Courbevoie (FR); RONDEAU, Christine, F-78500 Sartrouville (FR)
(74) Mandataire: Wattremez, Catherine
(86) Numéro de dépôt international: PCT/FR2001/003692
(87) Numéro de publication internationale: WO 2002/045673

(56) Documents cités:
- EP-A- 0 351 645
- WO-A-98/27942
- DE-A- 4 005 008

## Description

L'invention concerne une composition destinée à la teinture d'oxydation des fibres kératiniques, en particulier des fibres kératiniques humaines et plus particulièrement les cheveux, comprenant, dans un milieu approprié pour la teinture, au moins un colorant d'oxydation, au moins un alcool gras en C₁₂-C₃₀ et au moins un alcool gras en C₁₂-C₃₀ oxyalkyléné ou polyglycérolé et en outre 2 à 15% en poids et de préférence 5 à 10% en poids d'un mélange de glycérine (A) et d'un polyol différent de la glycérine (B) dans un rapport pondéral (A)/(B) > 1.
L'invention concerne également les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
La variété des molécules mises en jeu, qui sont constituées d'une part, par les bases d'oxydation et d'autre part, par les coupleurs, permet l'obtention d'une palette très riche en coloris.
Pour varier encore les nuances obtenues avec lesdits colorants d'oxydation, ou les enrichir de reflets, il arrive qu'on leur ajoute des colorants directs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Le compositions qui contiennent les colorants d'oxydation et qui, une fois mélangées avec un oxydant, sont appliquées sur les fibres pour obtenir ladite teinture d'oxydation décrite ci-dessus, renferment souvent des alcools gras. Ces alcools gras permettent d'épaissir les milieux de teinture mais présentent l'inconvénient de freiner la montée des colorants sur la fibre; par ailleurs ils peuvent être irritants pour le cuir chevelu et les compositions de teinture qui les contiennent ne sont pas toujours très stables.

Voici maintenant que la demanderesse a découvert, de façon tout à fait inattendue et surprenante, de nouvelles compositions contenant des colorants d'oxydation qui sont donc dépourvues d'agent oxydant (et que l'on conviendra d'appeler au sens de la présente invention, compositions colorantes), à base d'alcools gras, destinées à être mélangées avec un oxydant pour pouvoir teindre les fibres kératiniques par un procédé dit de teinture d'oxydation et qui présentent l'avantage d'être stables et non irritantes; ces compositions colorantes ne freinent pas la montée des colorants sur les fibres et permettent en conséquence d'obtenir par oxydation, des nuances puissantes.
Cette découverte est à la base de la présente invention.

La présente invention a ainsi pour objet une nouvelle composition pour la teinture d'oxydation des fibres kératiniques, en particulier des fibres kératiniques humaines, et plus particulièrement les cheveux, comprenant, dans un milieu approprié pour la teinture, au moins un colorant d'oxydation, au moins un alcool gras en C₁₂-C₃₀ et au moins un alcool gras en C₁₂-C₃₀ oxyalkyléné ou polyglycérolé et qui est caractérisée par le fait qu'elle renferme en outre 2 à 15% en poids et de préférence 5 à 10% en poids d'un mélange de glycérine (A) et d'un polyol différent de la glycérine (B) dans un rapport pondéral (A)/(B) > 1.

L'invention vise également un procédé de teinture des fibres kératiniques, en particulier des fibres kératiniques humaines et plus particulièrement les cheveux, consistant à appliquer sur les fibres au moins une composition colorante comprenant, dans un milieu approprié pour la teinture, au moins un colorant d'oxydation, au moins un alcool gras en C₁₂-C₃₀ et au moins un alcool gras en C₁₂-C₃₀ oxyalkyléné ou polyglycérolé et en outre 2 à 15% en poids d'un mélange de glycérine (A) et d'un polyol différent de la glycérine (B) dans un rapport pondéral (A)/(B) > 1, la couleur étant révélée à pH alcalin, neutre ou acide, à l'aide d'une composition oxydante contenant au moins un agent oxydant qui est mélangée juste au moment de l'emploi à la composition colorante ou qui est appliquée séquentiellement sans rinçage intermédiaire.

L'invention a également pour objet un dispositif à 2 compartiments avec un premier compartiment renfermant une composition colorante comprenant, dans un milieu approprié pour la teinture, au moins un colorant d'oxydation, au moins un alcool gras en C₁₂-C₃₀ et au moins un alcool gras en C₁₂-C₃₀ oxyalkyléné ou polyglycérolé et en outre 2 à 15% en poids d'un mélange de glycérine (A) et d'un polyol différent de la glycérine (B) dans un rapport pondéral (A)/(B) > 1, et un deuxième compartiment renfermant une composition oxydante comprenant, dans un milieu approprié pour la teinture, au moins un agent oxydant.

Mais d'autres caractéristiques, aspects, objets et avantages de l'invention apparaîtront encore plus clairement à la lecture de la description et des exemples qui suivent.

### Alcools gras en C₁₂-C₃₀, et alcools gras en C₁₂-C₃₀ oxyalkylénés ou polyglycérolés

Par alcool gras en C₁₂-C₃₀, on entend tout alcool gras pur, saturé ou non, ramifié ou non, ayant de 12 à 30 atomes de carbone ou des mélanges de ceux-ci.
A titre d'exemple d'alcools gras selon l'invention, on peut citer par exemple, l'alcool cétylique, l'alcool oléique, l'alcool stéarylique, l'alcool cétyl-stéarylique, l'alcool béhénique ou l'alcool érucique.
On peut également citer les produits commerciaux NAFOL 18-22®, NAFOL 18-22B®, NAFOL 18-22 C®, NAFOL 20+®,NAFOL 20-22®, NACOL 22-98® de la société CONDEA, le CRODACID PG 3220® de la société CRODA, l'EDENOR U 122 ® de la société HENKEL.

Les alcools gras en C₁₂-C₃₀ représentent en poids de 0,1 à 15% environ, de préférence de 0,5 à 10% environ du poids total de la composition.

Par alcool gras en C₁₂-C₃₀ oxyalkyléné ou polyglycérolé, on entend tout alcool gras pur oxyalkyléné ou polyglycérolé, saturé ou non, linéaire ou ramifié, dont le nombre de groupements oxyde d'éthylène ou oxyde de propylène est compris entre 2 et 100 et le nombre de groupements glycérol est compris entre 1 et 30 et de préférence entre 1 et 10, ou leurs mélanges.

Des alcools gras en C₁₂-C₃₀ oxyalkylénés particulièrement préférés selon l'invention sont des alcools gras saturés ou non, linéaires ou ramifiés, comportant 2 à 40 groupements oxyde d'éthylène.

Comme alcools gras oxyalkylénés, on peut notamment citer les produits commercialisés suivants :
Mergital LM2® (COGNIS) [alcool laurique 2 OE ];
Ifralan L12® (IFRACHEM) et Rewopal 12® (GOLDSCHMITDT) [alcool laurique 12 OE];
Brij 58 ® (UNIQUEMA) et Simulsol 58® (SEPPIC) [alcool cétylique 20 OE];
Eumulgin 05® (COGNIS) [alcool oleocétylique 5 OE];
Mergital OC30® (COGNIS) [alcool oleocétylique 30 OE];
Brij 72® (UNIQUEMA) [alcool stéarylique 2 OE];
Brij 76® (UNIQUEMA) [alcool stéarylique 10 OE];
Brij 78P® (UNIQUEMA) [alcool stéarylique 20 OE];
Brij 700® (UNIQUEMA) [alcool stéarylique 100 OE];
Eumulgin B1® (COGNIS) [alcool cétylstéarylique 12 OE];
Eumulgin L® (COGNIS) [alcool cétylique 9 OE et 2 OP];
Witconol APM® (GOLDSCHMIDT) [alcool myristique 3 OP].

Comme alcools gras polyglycérolés, on peut notamment citer l'alcool laurique à 4 moles de glycérol (nom INCI : POLYGLYCERYL-4 LAURYL ETHER), l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

Dans un produit commercial peuvent coexister plusieurs espèces d'alcools gras oxyalkylénés ou polyglycérolés sous forme d'un mélange.

Les alcools gras oxyalkylénés ou polyglycérolés représentent en poids de 0,1 à 15% environ, de préférence de 0,5 à 10% environ du poids total de la composition.

### Polyols différents de la glycérine

Par polyol, on désigne au sens de l'invention des chaînes hydrocarbonées saturées ou insaturées, linéaires, cycliques ou ramifiées en C₃-C₁₀ et portant au moins deux fonctions -OH sur la chaîne, ainsi que les polyalkylènes glycols.
Pour les αω-diols ramifiés ou non ramifiés, [αω s'entend, quand il s'agit de composés ramifiés, par rapport à l'enchaînement carboné le plus long], on peut citer par exemple, le 1,3 propane-diol ; le 1,4-butanediol ; le 1,5-pentanediol ; le 1,6-hexanediol ; le néopentylglycol (ou 2,3-diméthyl-1,3-propanediol).
Parmi les diols 1,2 - 1,4 - 1,5 - 2,3 - 2,4 - 2,5 - 2,6 - 2,7 - 2,8 - 3,4 - 3,5 - 3,6 ramifiés ou non ramifiés, on peut citer, le 2,5-hexanediol ; le 2,4-pentanediol (ou amylèneglycol) ; le 2-méthyl-2,4-pentanediol (ou hexylèneglycol) ; le 2,3-diméthyl-2,3-butanediol (ou pinacol).
Parmi les triols, on peut notamment citer le 1,2,4-butanetriol et le 1,2,6-hexanetriol.
Parmi les polyalkylène glycols, on peut citer le diéthylène glycol, le dipropylène glycol et les polyéthylène glycols.

Selon la présente invention, on préfère utiliser un diol, dont l'hexylèneglycol est plus particulièrement préféré.

Dans les compositions colorantes selon l'invention, la glycérine représente de 1,5 à 14% en poids environ du poids total de la composition et le ou les polyols différents de la glycérine représentent de 0,5 à 7% en poids environ du poids total de la composition.

Les colorants d'oxydation utilisables selon l'invention sont choisis parmi les bases d'oxydation et/ou les coupleurs.
De préférence les compositions selon l'invention contiennent au moins une base d'oxydation.

La nature de ces bases d'oxydation n'est pas critique. Elles peuvent notamment être choisies parmi les ortho- et para-phénylènediamines, les bases doubles, les ortho- et para- aminophénols, les bases hétérocycliques suivantes ainsi que les sels d'addition de tous ces composés avec un acide.

On peut notamment citer :

### - (I) les paraphénylènediamines de formule (I) suivante et leurs sels d'addition avec un acide :

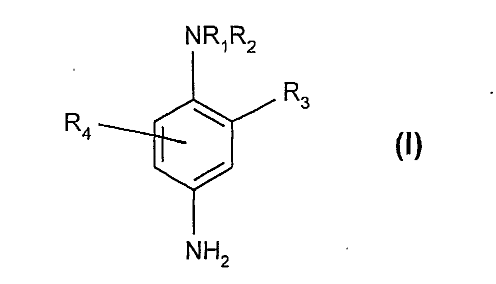

dans laquelle :
R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N, N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.

### -(II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 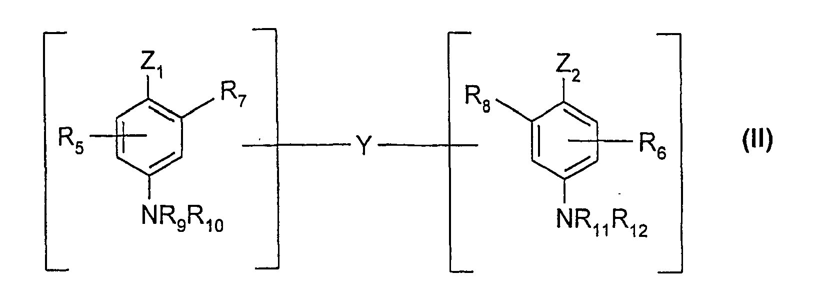 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle .en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
   étant entendu que les composés de formule (II) ne comportent qu'un seul bras de liaison Y par molécule.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.

### - (III) les para-aminophénols répondant à la formule (III) suivante, et leurs sels d'addition avec un acide :

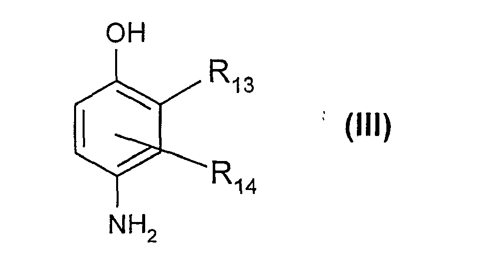

dans laquelle :
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénoi, le 4-amino-2-aminométhyl-phénol, le 4-amino-2-(β-hydroxyéthyl-aminométhyl)-phénol, et leurs sels d'addition avec un acide.

### - (IV) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.

### -(V) parmi les bases hétérocycliques utilisables à titre de bases d'oxydation dans les compositions colorantes conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

La composition colorante conforme à l'invention peut comprendre un ou plusieurs coupleurs choisis parmi ceux classiquement utilisés en teinture d'oxydation et notamment parmi les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénôl, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-inéthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Généralement le ou les coupleurs représentent de préférence de 0,0001 à 15% en poids environ du poids total de la composition colorante et encore plus préférentiellement de 0,001 à 10% environ.

Les sels d'addition avec un acide de ces colorants d'oxydation (bases et/ou coupleurs) sont notamment choisis parmi les chlorhydrates, les bromhydrates, les sulfates et les tartrates, les lactates et les acétates.

La composition colorante conforme à l'invention peut comprendre en outre un ou plusieurs colorants directs notamment pour modifier les nuances en les enrichissant de reflets. Ces colorants directs peuvent notamment être choisis parmi les colorants nitrés, azoïques ou anthraquinoniques, neutres, cationiques ou anioniques, classiquement utilisés ou ceux décrits notamment dans les demandes de brevet FR-2782450, 2782451, 2782452 et EP-1025834, dans la proportion pondérale d'environ 0,001 à 20% et de préférence de 0,01 à 10% du poids total de la composition.

Le milieu de la composition approprié pour la teinture, est de préférence un milieu aqueux constitué par de l'eau pouvant contenir avantageusement des solvants organiques acceptables sur le plan cosmétique et différents de (A) et de (B) selon la présente invention, dont plus particulièrement, des alcools tels que l'alcool éthylique, l'alcool isopropylique, l'alcool benzylique, et l'alcool phényléthylique, des éthers de glycol tels que, par exemple, les éthers monométhylique, monoéthylique et monobutylique d'éthylèneglycol, le monométhyléther de propylèneglycol, les alkyléthers de diéthylèneglycol comme par exemple, le monoéthyléther ou le monobutyléther du diéthylèneglycol. Les solvants peuvent alors être présents dans des concentrations allant d'environ 0,5 à 20% et, de préférence, d'environ 2 à 10% en poids par rapport au poids total de la composition.

La composition colorante selon l'invention peut en outre également comprendre des polymères associatifs.

### Polymères associatifs utilisés selon l'invention

Les polymères associatifs sont des polymères hydrosolubles capables, dans un milieu aqueux, de s'*associer* réversiblement entre eux ou avec d'autres molécules.
Leur structure chimique comprend des zones hydrophiles, et des zones hydrophobes caractérisées par au moins une chaîne grasse.
Les polymères associatifs selon l'invention peuvent être de type anionique, cationique, amphotère et de préférence non ionique.

### Polymères associatifs de type anionique :

On peut citer parmi eux :
- (**I**) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂=CR'CH₂OBₙR (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80® et SALCARE SC90® qui sont des émulsions aqueuses à 30% d'un terpolymère réticulé d'acide méthacrylique, d'acrylate d'éthyle et de steareth-10-allyl éther (40/50/10).
- (**II**) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 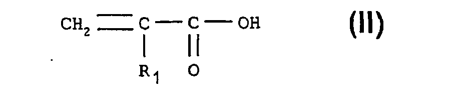 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante 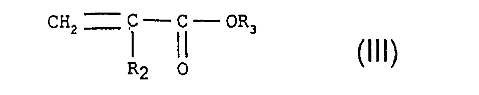 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères associatifs anioniques, on utilisera plus particulièrement des polymères formés à partir d'un mélange de monomères comprenant :
   (i) essentiellement de l'acide acrylique,
   (ii) un ester de formule (III) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
      Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment.
      Parmi lesdits polymères ci-dessus, on préfère tout particulièrement selon la présente invention, les produits vendus par la société GOODRICH sous les dénominations commerciales PEMULEN TR1®, PEMULEN TR2®, CARBOPOL 1382®, et encore plus préférentiellement le PEMULEN TR1®, et le produit vendu par la société S.E.P.P.I.C. sous la dénomination COATEX SX®.
- (**III**) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (**IV**) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique lacrylate de méthyle/diméthyl métaisopropényl benzyl isocyanate d'alcool béhényle éthoxylé (40OE) en dispersion aqueuse à 25%.
- (**V**) les copolymères comportant parmi leurs monomères un acide carboxylique à insaturation α,β-monoéthylénique et un ester d'acide carboxylique à insaturation α,β-monoéthylénique et d'un alcool gras oxyalkyléné.
   Préférentiellement ces composés comprennent également comme monomère un ester d'acide carboxylique à insaturation α,β-monoéthylénique et d'alcool en C1C4.
   A titre d'exemple de ce type de composé on peut citer l'ACULYN 22 ®vendu par la société ROHM et -HAAS, qui est un terpolymère acide méthacrylique/acrylate d'éthyle/méthacrylate de stéaryle oxyalkyléné.

### Polymères associatifs de type cationique

Selon la présente invention, ils sont choisis de préférence parmi les dérivés de cellulose quaternisée et les polyacrylates à groupements latéraux aminés non cycliques.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
   Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
   On peut indiquer comme exemples d'alkylhydroxyéthyl-celluloses quaternisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.

### Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon l'invention comprennent, ou sont préparés en copolymérisant :
1) au moins un monomère de formule (la) ou (Ib): 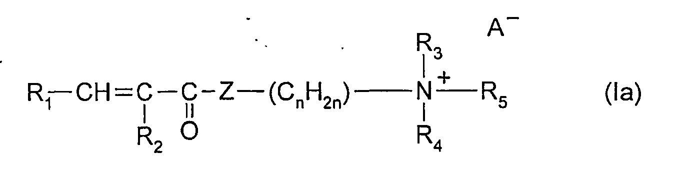 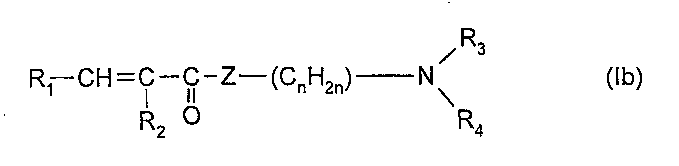 dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (II)

   R₆-CH= CR₇-COOH (II)

   dans laquelle, R₆ et R₇, identiques ou différents, représentent un atome d'hydrogène ou un radical méthyle;
   et
3) au moins un monomère de formule (III) :

   R₆-CH= CR₇- COXR₈ (III)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ;
   l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,
ces monomères étant éventuellement quaternisés, par exemple par un halogénure d'alkyle en C₁-C₄ ou un sulfate de dialkyle en C₁-C₄.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (la), (Ib) ou (III)), et de préférence de 1,5 à 6 moles % .

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères sont par exemple décrits et préparés dans la demande de brevet WO9844012.

Parmi les polymères associatifs amphotères selon l'invention, on préfère les terpolymères acide acrylique/chlorure de (méth)acrylamidopropyl triméthyl ammonium/ méthacrylate de stéaryle.

Dans la composition de teinture selon l'invention, on préfère utiliser un polymère associatif de type non ionique.

### Polymères associatifs de type non ionique

Selon l'invention, ils sont choisis de préférence parmi :
- (**1**) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (**2**) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (**3**) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone / hexadécène) vendu par la société I.S.P.
   - les produits ANTARON V220® ou GANEX V220® (copolymère vinylpyrrolidone / eicosène) vendu par la société I.S.P.
- (**4**) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
- (**6**) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
- (**7**) les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM®184.
On peut également citer le produit ELFACOS T210® à chaîne alkyle en C₁₂₋₁₄ et le produit ELFACOS T212® à chaîne alkyle en C₁₈ de chez AKZO.
Le produit DW 1206B de chez ROHM & HAAS à chaîne alkyle en C₂₀ et à liaison uréthanne, proposé à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Rhéolate 255®, le Rhéolate 278® et le Rhéolate 244® vendus par la société RHEOX. On peut aussi utiliser le produit DW 1206F et le DW 1206J proposés par la société ROHM & HAAS.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Les polymères associatifs de type non ionique, anionique, cationique ou amphotère sont utilisés de préférence en une quantité pouvant varier d'environ 0,1 à 10% en poids du poids total de la composition colorante. Plus préférentiellement, cette quantité varie d'environ 0,5 à 5% en poids, et encore plus particulièrement d'environ 1 à 3% en poids.

La composition colorante selon l'invention peut également contenir des agents tensio-actifs différant des alcools gras en C₁₂-C₃₀ oxyalkylénés ou polyglycérolés selon l'ivention et choisis parmi ceux de type anionique, non ionique, cationique, amphotère ou zwittérionique, dans la proportion d'au moins 0,01% en poids par rapport au poids total de la composition.

Ces agents tensio-actifs peuvent être choisis parmi :

### Les tensioactifs non ioniques :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative), les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine.

### Les tensioactifs anioniques :

A titre d'exemple de tensio-actifs anioniques utilisables, seuls ou mélanges; dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, a-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### Les tensioactifs amphotères ou zwitterioniques:

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.
Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO-)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{Z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### Les tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

La composition colorante selon l'invention peut en outre contenir, au moins un polymère cationique ou amphotère (différent des polymères associatifs décrits ci-dessus) dans la proportion d'au moins 0,01 % en poids par rapport au poids total de la composition.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :
(**1**) Les homopolymères ou copolymères dérivés d'esters ou d'amides acryliques ou méthacryliques et comportant au moins un des motifs de formules (I), (II), (III) ou (IV) suivantes: 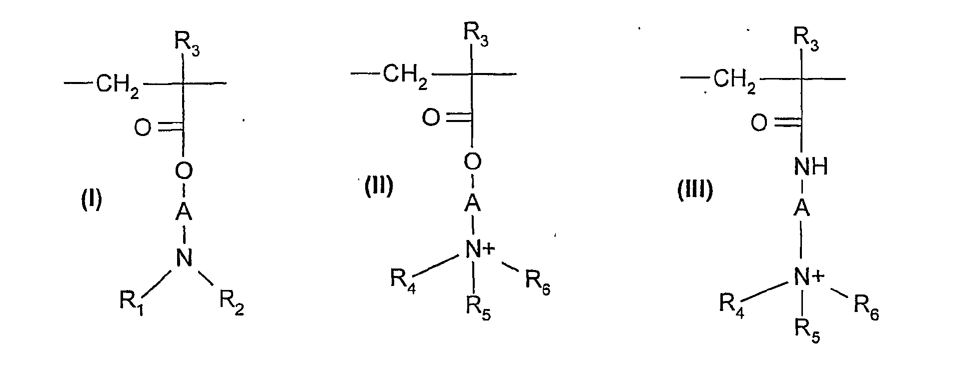 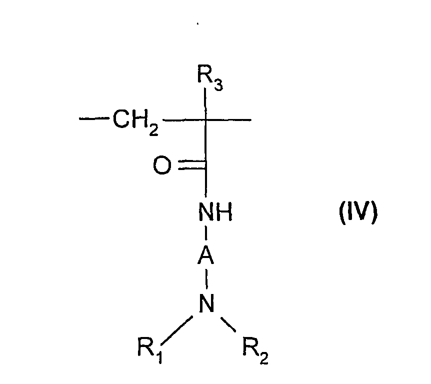 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un hologénure de diméthyle, tel que celui vendu sous la dénomination HERCOFLOC® par la société HERCULES,
   - les copolymères d'acrylamide et de chlorure de méthacryloyloxyéthyltriméthylammonium décrits par exemple dans la demande de brevet EP-A-080976 et vendus sous la dénomination BINA QUAT P 100® par la société CIBA GEIGY,
   - le copolymère d'acrylamide et de méthosulfate de méthacryloyloxyéthyltriméthylammonium vendu sous la dénomination RETEN® par la société HERCULES,
   - les copolymères vinylpyrrolidone / acrylate ou méthacrylate de dialkylaminoalkyle quaternisés ou non, tels que les produits vendus sous la dénomination "GAFQUAT®" par la société ISP comme par exemple "GAFQUAT 734" ou "GAFQUAT 755" ou bien les produits dénommés "COPOLYMER 845®, 958® et 937®". Ces polymères sont décrits en détail dans les brevets français 2.077.143 et 2.393.573,
   - les terpolymères méthacrylate de diméthyl amino éthyle/ vinylcaprolactame/vinylpyrrolidone tel que le produit vendu sous la dénomination GAFFIX VC 713® par la société ISP,
   - les copolymère vinylpyrrolidone / méthacrylamidopropyl dimethylamine commercialisés notamment sous la dénomination STYLEZE CC 10® par ISP,
   - et les copolymères vinylpyrrolidone / méthacrylamide de diméthylaminopropyle quaternisés tel que le produit vendu sous la dénomination "GAFQUAT HS 100®" par la société ISP.
**(2)** Les dérivés d'éthers de cellulose comportant des groupements ammonium quaternaire décrits dans le brevet français 1 492 597, et en particulier les polymères commercialisés sous les dénominations "JR®" (JR 400, JR 125, JR 30M) ou "LR®" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
**(3)** Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium. Les produits commercialisés répondant à cette définition sont plus particulièrement les produits vendus sous la dénomination "Celquat L 200®" et "Celquat H 100®" par la Société National Starch.
**(4)** Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S®, JAGUAR C 15®, JAGUAR C 17® ou JAGUAR C162® par la société MEYHALL.
**(5)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280,361.
**(6)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
**(7)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine® F, F4 ou F8" par la société Sandoz.
**(8)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57®" par la société Hercules Inc. ou bien sous la dénomination de "PD 170®" ou "Delsette 101®" par la société Hercules dans le cas du copolymère d'acide adipique/époxypropyl/diéthylène-triamine.
**(9)** Les cyclopolymères de dialkyl diallyl ammonium tels que les homopolymères ou copolymères comportant comme constituant principal de la chaîne des motifs répondant à la formule (V) : 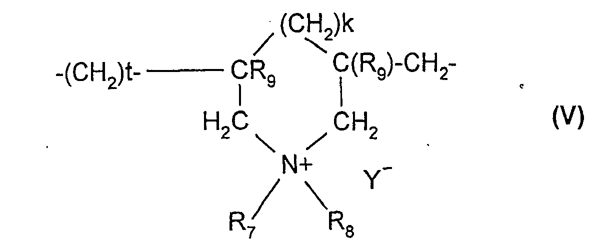 dans laquelle k et t sont égaux à 0 ou 1, la somme k + t étant égale à 1 ;
   R₇ et R₈, identiques ou différents, désignent un groupement alkyle ayant de 1 à 8 atomes de carbone, un groupement hydroxyalkyle(C₁-C₅), un groupement amidoalkyle(C₁-C₄), ou R₇ et R₈ désignent conjointement avec l'atome d'azote auquel ils sont rattachés, un groupement pipéridinyle ou morpholinyle ;
   Rg désigne un atome d'hydrogène ou un radical méthyle ;
   R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100®" par la société Calgon (et ses homologues de faible masse moléculaire moyenne en poids) et les copolymères de chlorure de diallyldiméthylammonium et d'acrylamide commercialisés sous la dénomination "MERQUAT 550®".
**(10)** Le polymère de diammonium quaternaire contenant des motifs récurrents répondant à la formule : 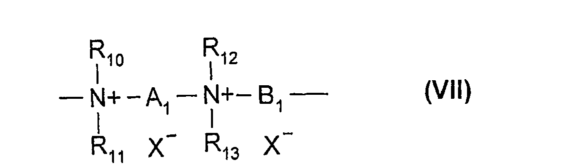 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH-R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement - (CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :
         - (CH₂-CH₂-O)x-CH₂-CH₂-
         - [CH₂-CH(CH₃)-O]y-CH₂-CH(CH₃)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent
         -CH₂-CH₂-S-S-CH₂-CH₂- ;
      d) un groupement uréylène de formule : -NH-CO-NH-.
      De préférence, X⁻ est un anion tel que le chlorure ou le bromure. Ces polymères ont une masse moléculaire moyenne en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

   On peut utiliser plus particulièrement les polymères qui sont constitués de motifs récurrents répondant à la formule (VIII) suivante: 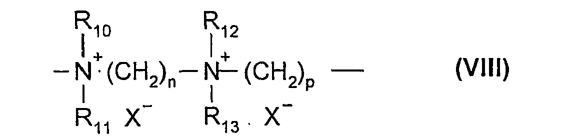 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(11) Les polymères de polyammonium quaternaire constitués de motifs récurrents de formule (IX) : 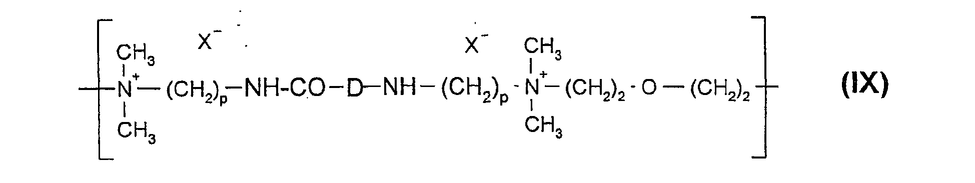 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15®", "Mirapol AD1®", "Mirapol AZ1®" et "Mirapol 175®" vendus par la société Miranol.
**(12)** Les polymères quaternaires de vinylpyrrolidone et de vinylimidazole tels que par exemple les produits commercialisés sous les dénominations Luviquat FC 905®, FC 550® et FC 370® par la société B.A.S.F.
**(13)** Les polyamines comme le Polyquart H® vendu par HENKEL, référencé sous le nom de " POLYETHYLENEGLYCOL (15) TALLOW POLYAMINE " dans le dictionnaire CTFA.
**(14)** Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (9), (10) et (11) et encore plus préférentiellement les suivants :
-1/ l'homopolymère de chlorure de diméthyldiallylammonium (famille 9);
-2/ les polymères aux motifs récurrents de formule (VIII) décrite ci-avant (famille 10) et pour laquelle
   R₁₀, R₁₁, R₁₂ et R₁₃ représentent un radical méthyle, n = 3, p = 6 et X = Cl, et notamment ceux dont le poids moléculaire, déterminé par chromatographie par perméation de gel, est compris entre 9500 et 9900 [Polymère W de formule ci-dessous]; R₁₀ et R₁₁ représentent un radical méthyle, R₁₂ et R₁₃ représentent un radical éthyle et n = p = 3 et X = Br, et notamment ceux dont le poids moléculaire, déterminé par chromatographie par perméation de gel, est d'environ 1200 [Polymère U de formule ci-dessous]. 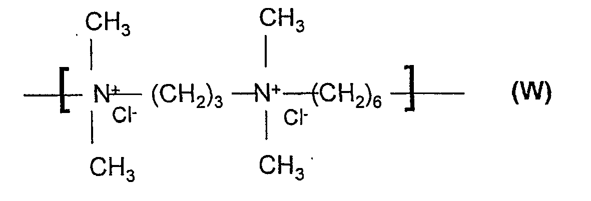 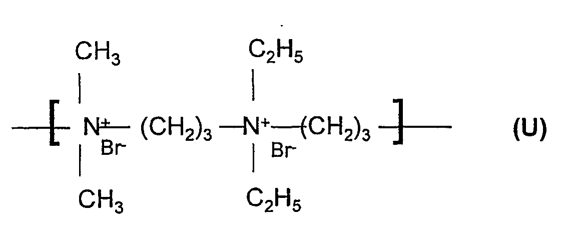 Lesdits polymères à motifs (W) et (U) sont préparés et décrits dans le brevet français 2 270 846.
-3/ les polymères à motifs de formule (IX) décrite ci-dessus (famille 11), pour laquelle, p est égal à 3, et,
   a) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15®,
   b) D représente un groupement -(CH₂)₄ -CO -, X désigne un atome de chlore, la masse moléculaire mesurée par RMN du Carbone 13 ( RMN¹³C ) étant d'environ 5600 ; un polymère de ce type est proposé par la société MIRANOL sous le nom de MIRAPOL-AD1®,
   c) D représente un groupement -(CH₂)₇ -CO-, X désigne un atome de chlore, la masse moléculaire mesurée par RMN du Carbone 13 ( RMN¹³C) étant d'environ 8100 ; un polymère de ce type est proposé par la société MIRANOL sous le nom de MIRAPOL-AZ1®,
   d) un " Block Copolymer " formé de motifs correspondant aux polymères décrits aux alinéas a) et b), proposé par la société MIRANOL sous les noms MIRAPOL-9®, (masse moléculaire RMN¹³C, environ 7800) MIRAPOL-175®, (masse moléculaire RMN¹³C, environ 8000) MIRAPOL-95®, (masse moléculaire RMN¹³C, environ 12500). Plus particulièrement encore, on préfère selon l'invention, le polymère à motifs de formule (IX) dans laquelle p est égal à 3, D désigne la valeur zéro, X désigne un atome de chlore, la masse moléculaire mesurée par RMN du Carbone 13 ( RMN¹³C) étant d'environ 25500 (Mirapol-A15®).

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminaalkylméthacrylamide et acrylamide. De tels composés sont décrits dans le brevet américain n° 3 836 537.
   On peut également citer le copolymère acrylate de sodium / chlorure d'acrylamidopropyl trimethyl ammonium vendu sous la dénomination POLYQUART KE 3033® par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appelations MERQUAT 280® et MERQUAT 295®, par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
      Les acrylamides ou méthacrylamides N-substitués plus particulièrement préférés selon l'invention sont les groupements dont les radicaux alkyle contiennent dé 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle.
      On utilise particulièrement les copolymères dont la dénomination CTFA (4ème Ed., 1991) est Octylacrylamide/acrylates/butylaminoethylmethacryfate copolymer tels que les produits vendus sous la dénomination AMPHOMER® ou LOVOCRYL 47® par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent.dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono
   ou bis-secondaire et de préférence représente :
   a) dans les proportions de 60 à 100 moles %, le radical 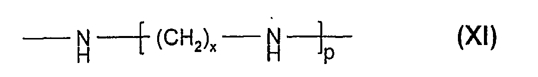 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 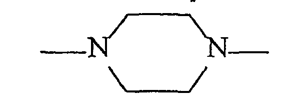
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
      Les alcanes sultones utilisées dans l'alcoylation sont de préférence la propane ou la butane sultone, les sels des agents d'alcoylation sont de préférence les sels de sodium ou dé potassium.
(4) Les polymères comportant des motifs zwittérioniques de formule : 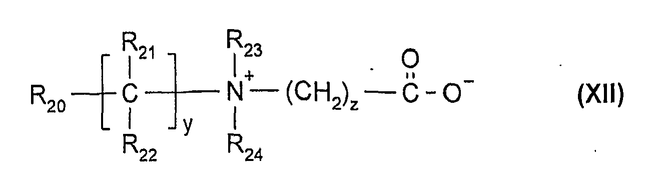 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301® par la société SANDOZ.
(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne: 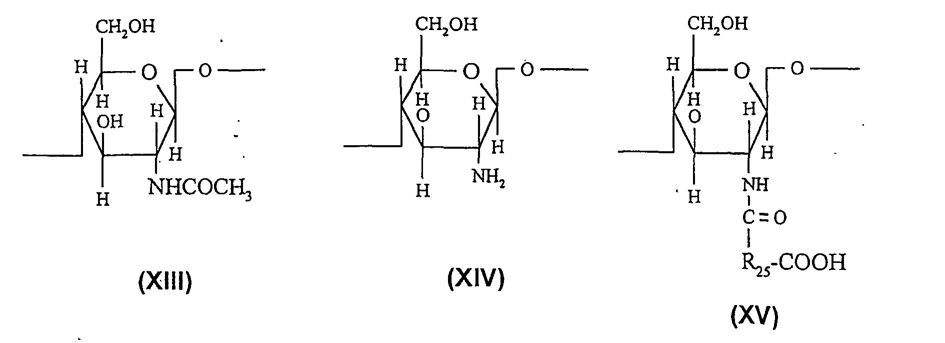 le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), R₂₅ représente un radical de formule : 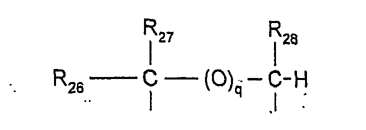 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
   Des polymères de ce type plus particulièrement préférés comportent de 0 à 20% en poids de motifs (XIII), de 40 à 50% en poids de motifs (XIV), et de 40 à 50% en poids de motifs (XV) dans lequel R₂₅ désigne le radical -CH₂-CH₂- ;
(6) Les polymères dérivés de la N-carboxyalkylation du chitosane comme le N-carboxyméthyl chitosane ou le N-carboxybutyl chitosane vendu sous la dénomination "EVALSAN®" par la société JAN DEKKER.
(7) Les polymères répondant à la formule générale (XI) tels que ceux décrits par exemple dans le brevet français 1 400 366 : 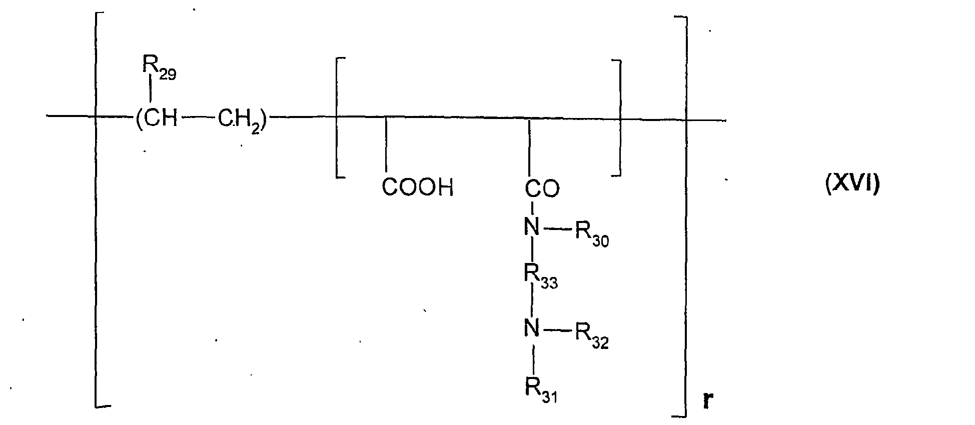 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂-, -CH₂-CH(CH₃)-, R₃₁ ayant les significations mentionnées ci-dessus, ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 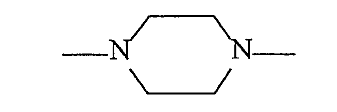 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)

      où D désigne un radical 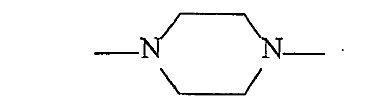 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères amphotères susceptibles d'être utilisés dans le cadre de la présente invention, on préfère mettre en oeuvre les polymères de la famille (1), et en particulier les copolymères d'acide acrylique et de chlorure de diméthyldiallylammonium.

Selon l'invention, le ou les polymères cationiques ou amphotères différant des polymères associatifs cationiques ou amphotères, peuvent représenter environ 0,01 % à 10 % en poids, de préférence 0,05 % à 5 % en poids, et encore plus préférentiellement 0,1 % à 3 % en poids, du poids total de la composition.

La composition colorante peut encore comprendre une quantité efficace d'autres agents, par ailleurs antérieurement connus en teinture d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ladite composition peut également comprendre des agents réducteurs ou antioxydants. Ceux-ci peuvent être choisis en particulier parmi le métabisulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

Ladite composition peut également comprendre d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques tels que les homopolymères réticulés d'acide acrylique ou d'acide acrylamidopropanesulfonique.
Ces épaississants d'appoint peuvent représenter de 0,01 à 10% en poids du poids total de la composition.

Bien entendu, l'homme de l'art veillera à choisir le ou les éventuels composés complémentaires mentionnés ci-avant, de manière telle que les propriétés avantageuses attachées intrinsèquement à la composition selon l'invention ne soient pas, ou substantiellement pas, altérées par la ou les adjonctions envisagées.

Le procédé de teinture selon l'invention consiste, de préférence, à appliquer un mélange, réalisé extemporanément au moment de l'emploi, à partir de la composition colorante selon l'invention et d'une composition oxydante, sur les fibres kératiniques sèches ou humides, et à le laisser agir pendant un temps de pause variant, de préférence, de 1 à 60 minutes environ, et plus préférentiellement de 5 à 45 minutes environ, à rincer les fibres, puis éventuellement à les laver au shampooing, puis à les rincer à nouveau, et à les sécher.
Dans la composition oxydante, l'agent oxydant est choisi de préférence parmi le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les oxydoréductases à 4 électrons (comme les laccases), les peroxydases et les oxydoréductases à 2 électrons (telles que l'uricase), le cas échéant en présence de leur donneur ou cofacteur respectif.'
Le pH de la composition appliquée sur les fibres kératiniques [composition résultant du mélange de la composition colorante selon l'invention et de la composition oxydante], est généralement compris entre les valeurs 3 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (VI) suivante : 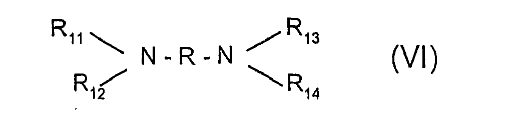 dans laquelle R est un reste propylène éventuellement substitué par un groupement hydroxyle ou un radical alkyle en C₁-C₄ ; R₁₁, R₁₂, R₁₃ et R₁₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Des exemples concrets illustrant l'invention vont maintenant être donnés, sans pour autant présenter un caractère limitatif.

### EXEMPLES:

On a préparé la composition colorante, conforme à l'invention, suivante :
(exprimée en grammes)

| | |
|---|---|
| Mélange d'alcools linéaires en C18 à C24 [C18/C20/C22/C24 : 7/58/30/6 -teneur en alcool > 95%] | 3 |
| Mélange d'alcools linéaires en C18 à C24 [C18/C20/C22/C24 : 7/58/30/6 -teneur en alcool > 95%] oxyéthylénés (30 OE) | 1 |
| Alcool stéarylique oxyéthyléné (2 OE) | 4,5 |
| Alcool stéarylique oxyéthyléné (21 OE) | 1,75 |
| Acide polyacrylique réticulé (Carbopol 980 de GOODRICH) | 0,6 |
| Acide oléique | 2,6 |
| Aculyn 44 ou Aculyn 46 vendu par ROHM & HAAS | 4 |
| Monoisopropanolamide d'acides de coprah | 3 |
| Polymère cationique W selon l'invention | 4 MA^{*} |
| Glycérine | 5 |
| Hexylène glycol | 1 |
| Métabisulfite de sodium | 0,71 |
| EDTA (Acide éthylènediamine tétra-acétique) | 0,2 |
| Ter-butyl hydroquinone | 0,3 |
| 1,4-diaminobenzène | 0,2 |
| Para-aminophénol | 1 2 |
| 1,3-dihydroxybenzène | 0,1 |
| 1-hydroxy-3-amino-benzène | 0,2 |
| 1-méthyl-2-hydroxy-4-β-hydroxyéthylamino-benzène | 0,8 |
| Monoéthanolamine | 1 |
| Ammoniaque à 20% de NH3 | 11 |
| Parfum q.s | |
| Eau déminéralisée q.s.p | 100 |

| | |
|---|---|
| MA^{*} désigne Matière Active | |

| | |
|---|---|
| Composition oxydante : | |
| Alcool gras | 2,3 |
| Alcool gras oxyéthyléné | 0,6 |
| Amide grasse | 0,9 |
| Glycérine | 0,5 |
| Peroxyde d'hydrogène | 7,5 |
| parfum | qs |
| Eau déminéralisée qsp | 100 |

Ladite composition colorante a été mélangée, au moment de l'emploi, dans un bol en plastique à la composition oxydante donnée ci-dessus, à raison de 1 partie de composition colorante pour 1,5 parties de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing standard, à nouveau rincées à l'eau, puis séchées et démélées.
Les cheveux ont été teints dans une nuance soutenue chatain clair rouge cuivré, chromatique et tenace et l'état cosmétique du cheveu a été amélioré.

Des résultats identiques ont été obtenus en remplaçant, dans la composition colorante ci-dessus, l'hexylène glycol par la même quantité de 1,5-pentanediol.

## Revendications

1. Composition pour la teinture d'oxydation des fibres kératiniques, en particulier des fibres kératiniques humaines, et plus particulièrement les cheveux, comprenant, dans un milieu approprié pour la teinture, au moins un colorant d'oxydation, au moins un alcool gras en C₁₂-C₃₀, au moins un alcool gras en C₁₂-C₃₀ oxyalkyléné ou polyglycérolé, et qui est **caractérisée par le fait qu'**elle renferme en outre 2 à 15% en poids d'un mélange de glycérine (A) et d'un polyol différent de la glycérine (B) dans un rapport pondéral (A)/(B) > 1.

2. Composition selon la revendication 1, **caractérisée par le fait que** le mélange de glycérine (A) et de polyol différent de la glycérine (B) est présent dans des concentrations comprises entre 5 et 10% en poids par rapport au poids total de la composition.

3. Composition selon la revendication 1 ou 2, **caractérisée par le fait que** le polyol différent de la glycérine est un diol.

4. Composition selon la revendication 3, **caractérisée par le fait que** le diol est l'hexylène glycol.

5. Composition selon l'une quelconque des revendications précédentes, **caractérisée par le fait que** la glycérine est présente dans des concentrations comprises entre 1,5 et 14% en poids par rapport au poids total de la composition.

6. Composition selon l'une quelconque des revendications précédentes, **caractérisée par le fait que** le ou les polyols différents de la glycérine sont présents dans des concentrations comprises entre 0,5 et 7% en poids par rapport au poids total de la composition.

7. Composition selon l'une quelconque des revendications précédentes, **caractérisée par le fait que** le ou les alcools gras en C₁₂-C₃₀ sont présents dans des concentrations comprises entre 0,1 et 15% en poids par rapport au poids total de la composition.

8. Composition selon la revendication 7, **caractérisée par le fait que** le ou les alcools gras en C₁₂-C₃₀ sont présents dans des concentrations comprises entre 0,5 et 10% en poids par rapport au poids total de la composition.

9. Composition selon l'une quelconque des revendications précédentes, **caractérisée par le fait que** le ou les alcools gras en C₁₂-C₃₀ oxyalkylénés ou polyglycérolés sont présents dans des concentrations comprises entre 0,1 et 15% en poids par rapport au poids total de la composition.

10. Composition selon la revendication 9, **caractérisée par le fait que** le ou les alcools gras en C₁₂-C₃₀ oxyalkylénés ou polyglycérolés sont présents dans des concentrations comprises entre 0,5 et 10% en poids par rapport au poids total de la composition.

11. Composition selon l'une quelconque des revendications précédentes, **caractérisée par le fait que** le ou les colorants d'oxydation sont choisis parmi les bases d'oxydation et/ou les coupleurs.

12. Composition selon l'une quelconque des revendications précédentes, **caractérisée par le fait qu'**elle renferme au moins une base d'oxydation.

13. Composition selon la revendication 12, **caractérisée par le fait que** les bases d'oxydation sont choisies parmi les ortho- ou para-phénylènediamines, les bases doubles, les ortho- ou para-aminophénols, et les bases hétérocycliques, ainsi que les sels d'addition de ces composés avec un acide, et sont présentes dans des concentrations allant de 0,0005 à 12% en poids par rapport au poids total de la composition.

14. Composition selon la revendication 11, **caractérisée par le fait que** les coupleurs sont choisis parmi les méta-aminophénols, les métaphénylènediamines, les métadiphénols, les naphtols, les coupleurs hétérocycliques, et les sels d'addition de ces composés avec un acide, et sont présents dans des concentrations allant de 0,0001 à 15% en poids par rapport au poids total de la composition.

15. Composition selon l'une quelconque des revendications 11 à 14, **caractérisée par le fait que** les sels d'addition avec un acide des colorants d'oxydation (bases et coupleurs) sont choisis parmi les chlorhydrates, les bromhydrates, les sulfates, les tartrates, les lactates et les acétates.

16. Composition selon l'une quelconque des revendications précédentes, **caractérisée par le fait qu'**elle comprend en outre au moins un colorant direct dans la proportion de 0,001 à 20% en poids par rapport au poids total de la composition.

17. Composition selon l'une quelconque des revendications précédentes, **caractérisée par le fait que** le milieu approprié pour la teinture est un milieu aqueux ou un milieu eau et solvant organique différent de (A) et de (B).

18. Composition selon la revendication 17, **caractérisée par le fait que** les solvants organiques différents de (A) et de (B) sont présents dans des concentrations allant de 0,5 à 20% en poids par rapport au poids total de la composition.

19. Composition selon l'une quelconque des revendications précédentes, **caractérisée par le fait qu'**elle comprend au moins un polymère associatif anionique, non ionique, cationique ou amphotère.

20. Composition selon la revendication 19, **caractérisée par le fait que** le ou les polymères associatifs sont non ioniques et choisis parmi les polyéthers polyuréthanes.

21. Composition selon la revendication 20, **caractérisée par le fait que** le polyéther polyuréthane est un polycondensat de polyéthylèneglycol à 150 ou 180 moles d'oxyde d'éthylène, d'alcool stéarylique et d'au moins un diisocyanate.

22. Composition selon la revendication 21, **caractérisée par le fait qu'**il s'agit d'un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81 %).

23. Composition selon la revendication 20, **caractérisée par le fait que** le polyéther polyuréthane est un polycondensat de polyéthylèneglycol à 150 ou 180 moles d'oxyde d'éthylène, d'alcool décylique et d'au moins un diisocyanate.

24. Composition selon la revendication 23, **caractérisée par le fait que** le polyéther polyuréthane est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

25. Composition selon l'une quelconque des revendications 19 à 24, **caractérisée par le fait que** le ou les polymères associatifs sont présents dans la proportion de 0,1 à 10% en poids par rapport au poids total de la composition.

26. Composition selon l'une quelconque des revendications précédentes, **caractérisée par le fait qu'**elle comprend en outre au moins un polymère cationique ou amphotère différant des polymères associatifs décrits à l'une quelconque des revendications 19 à 25.

27. Composition selon la revendication 26, **caractérisée par le fait que** le polymère cationique ou amphotère est choisi parmi :
-1/ les homopolymères de chlorure de diméthyldiallylammonium;
-2/ les polymères au motifs récurrents de formules (W) ou (U) suivantes : 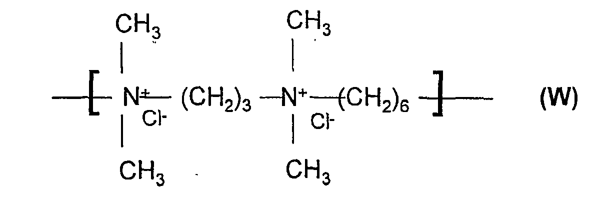 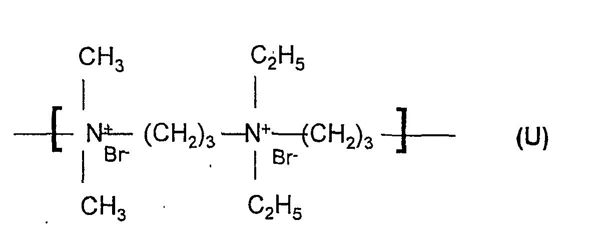
-3/ les polymères aux motifs de formule (IX) suivante : 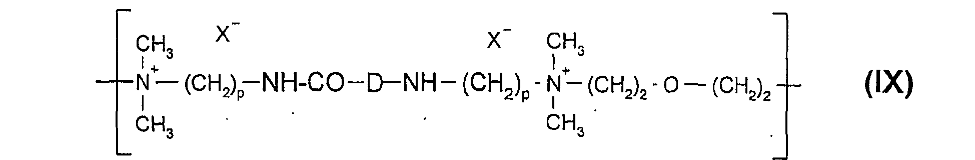 pour laquelle, p est égal à 3, et,
a) D désigne la valeur zéro, X désigne un atome de chlore,
b) D représente un groupement -(CH₂)₄ -CO-, X désigne un atome de chlore,
c) D représente un groupement -(CH₂)₇-CO-, X désigne un atome de chlore,
d) un " Block Copolymer " formé de motifs correspondant aux polymères décrits aux alinéas a) et b);
-4/ les copolymères d'acide acrylique et de chlorure de diméthyldiallylammonium.

28. Composition selon la revendication 26 ou 27, **caractérisée par le fait que** le ou lesdits polymères cationiques ou amphotères sont présents dans la proportion de 0,01 à 10% en poids par rapport au poids total de la composition.

29. Composition selon l'une quelconque des revendications précédentes, **caractérisée par le fait qu'**elle comprend au moins un agent tensioactif différant des alcools gras en C₁₂-C₃₀ oxyalkylénés ou polyglycérolés choisi parmi les tensioactifs anioniques, cationiques, non ioniques, amphotères ou zwittérioniques dans la proportion d'au moins 0,01% en poids par rapport au poids total de la composition.

30. Procédé de teinture des fibres kératiniques, en particulier des fibres kératiniques humaines et plus particulèrement les cheveux, **caractérisé par le fait qu'**il consiste à appliquer sur les fibres au moins une composition colorante telle que définie à l'une quelconque de revendications 1 à 29, la couleur étant révélée à pH alcalin, neutre ou acide à l'aide d'une composition oxydante comprenant au moins un agent oxydant, qui est mélangée juste au moment de l'emploi à la composition colorante ou qui est appliquée séquentiellement sans rinçage intermédiaire.

31. Procédé selon la revendication 30, **caractérisé par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

32. Procédé selon la revendication 31, **caractérisé par le fait que** l'agent oxydant est une solution d'eau oxygénée dont le titre varie de 1 à 40 volumes.

33. Procédé selon l'une quelconque des revendications 30 à 32, **caractérisé par le fait que** le pH est compris entre 6 et 11.

34. Procédé selon l'une quelconque des revendications 30 à 33, **caractérisé par le fait qu'**il consiste à appliquer sur les fibres kératiniques sèches ou humides, la composition réalisée extemporanément au moment de l'emploi à partir des compositions colorante et oxydante, à la laisser agir pendant un temps de pause variant de 1 à 60 minutes environ, et de préférence de 5 à 45 minutes, à rincer les fibres, puis éventuellement à les laver au shampooing, puis à les rincer à nouveau, et à les sécher.

35. Dispositif à 2 compartiments ou « Kit » pour la teinture des fibres kératiniques, en particulier des fibres kératiniques humaines et plus particulièrement les cheveux, **caractérisé par le fait que** le premier compartiment renferme une composition colorante telle que définie à l'une quelconque des revendications 1 à 29, et le deuxième compartiment renferme une composition oxydante.

## Claims

1. Composition for the oxidation dyeing of keratinous fibres, in particular human keratinous fibres, and more particularly hair, comprising, in an appropriate medium for dyeing, at least one oxidation dye, at least one C₁₂-C₃₀ fatty alcohol and at least one oxyalkylenated or polyglycerolated C₁₂-C₃₀ fatty alcohol and which is **characterized in that** it additionally contains 2 to 15% by weight of a mixture of glycerine (A) and of a polyol different from glycerine (B) in an (A)/(B) weight ratio > 1.

2. Composition according to Claim 1, **characterized in that** the mixture of glycerine (A) and polyol different from glycerine (B) is present in concentrations between 5 and 10% by weight relative to the total weight of the composition.

3. Composition according to Claim 1 or 2, **characterized in that** the polyol different from glycerine is a diol.

4. Composition according to Claim 3, **characterized in that** the diol is hexylene glycol.

5. Composition according to any one of the preceding claims, **characterized in that** the glycerine is present in concentrations between 1.5 and 14% by weight relative to the total weight of the composition.

6. Composition according to any one of the preceding claims, **characterized in that** the polyol(s) different from glycerine is/are present in concentrations between 0.5 and 7% by weight relative to the total weight of the composition.

7. Composition according to any one of the preceding claims, **characterized in that** the C₁₂-C₃₀ fatty alcohol(s) is/are present in concentrations between 0.1 and 15% by weight relative to the total weight of the composition.

8. Composition according to Claim 7, **characterized in that** the C₁₂-C₃₀ fatty alcohol (s) is/are present in concentrations between 0.5 and 10% by weight relative to the total weight of the composition.

9. Composition according to any one of the preceding claims, **characterized in that** the oxyalkylenated or polyglycerolated C₁₂-C₃₀ fatty alcohol(s) is/are present in concentrations between 0.1 and 15% by weight relative to the total weight of the composition.

10. Composition according to Claim 9, **characterized in that** the oxyalkylenated or polyglycerolated C₁₂-C₃₀ fatty alcohol(s) is/are present in concentrations between 0.5 and 10% by weight relative to the total weight of the composition.

11. Composition according to any one of the preceding claims, **characterized in that** the oxidation dye(s) is/are chosen from oxidation bases and/or couplers.

12. Composition according to any one of the preceding claims, **characterized in that** it contains at least one oxidation base.

13. Composition according to Claim 12, **characterized in that** the oxidation bases are chosen from ortho- or para-phenylenediamines, double bases, ortho- or para-amiriophenols, and heterocyclic bases, and the addition salts of these compounds with an acid, and are present in concentrations ranging from 0.0005 to 12% by weight relative to the total weight of the composition.

14. Composition according to Claim 11, **characterized in that** the couplers are chosen from meta-aminophenols, meta-phenylenediamines, meta-diphenols, naphthols, heterocyclic couplers, and the addition salts of these compounds with an acid, and are present in concentrations ranging from 0.0001 to 15% by weight relative to the total weight of the composition.

15. Composition according to any one of Claims 11 to 14, **characterized in that** the addition salts with an acid of the oxidation dyes (bases and couplers) are chosen from hydrochlorides, hydrobromides, sulphates, tartrates, lactates and acetates.

16. Composition according to any one of the preceding claims, **characterized in that** it additionally comprises at least one direct dye in the proportion of 0.001 to 20% by weight relative to the total weight of the composition.

17. Composition according to any one of the preceding claims, **characterized in that** the appropriate medium for dyeing is an aqueous medium or a water and organic solvent medium different from (A) and from (B).

18. Composition according to Claim 17, **characterized in that** the organic solvents different from (A) and from (B) are present in concentrations ranging from 0.5 to 20% by weight relative to the total weight of the composition.

19. Composition according to any one of the preceding claims, **characterized in that** it comprises at least one anionic, nonionic, cationic or amphoteric associative polymer.

20. Composition according to Claim 19, **characterized in that** the associative polymer(s) is/are nonionic and are chosen from polyether-polyurethanes.

21. Composition according to Claim 20, **characterized in that** the polyether-polyurethane is a polycondensate of polyethylene glycol containing 150 or 180 mol of ethylene oxide, stearyl alcohol and at least one diisocyanate.

22. Composition according to Claim 21, **characterized in that** it is a polycondensate of polyethylene glycol containing 150 or 180 mol of ethylene oxide, stearyl alcohol and methylenebis(4-cyclohexyl isocyanate) (SMDI), at 15% by weight in a matrix of maltodextrin (4%) and water (81%).

23. Composition according to Claim 20, **characterized in that** the polyether-polyurethane is a polycondensate of polyethylene glycol containing 150 or 180 mol of ethylene oxide, decyl alcohol and at least one diisocyanate.

24. Composition according to Claim 23, **characterized in that** the polyether-polyurethane is a polycondensate of polyethylene glycol containing 150 or 180 mol of ethylene oxide, decyl alcohol and methylenebis(4-cyclohexyl isocyanate) (SMDI), at 35% by weight in a mixture of propylene glycol (39%) and water (26%).

25. Composition according to any one of Claims 19 to 24, **characterized in that** the associative polymer(s) is/are present in the proportion of 0.1 to 10% by weight relative to the total weight of the composition.

26. Composition according to any one of the preceding claims, **characterized in that** it additionally comprises at least one cationic or amphoteric polymer different from the associative polymers described in any one of Claims 19 to 25.

27. Composition according to Claim 26, **characterized in that** the cationic or amphoteric polymer is chosen from:
-1/ the homopolymers of dimethyldiallylammonium chloride;
-2/ the polymers containing the repeating units of the following formulae (W) or (U): 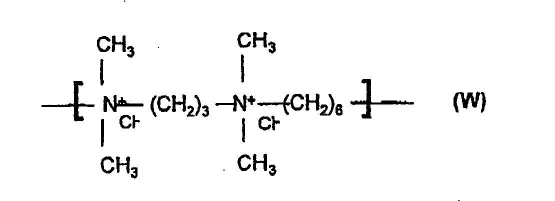 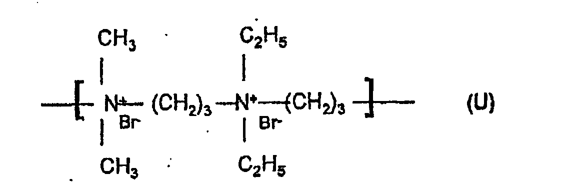
-3/ the polymers containing the units of the following formula (IX) : 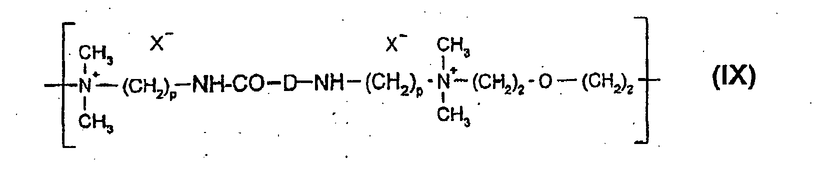 for which p is equal to 3, and,
a) D denotes the value zero, X denotes a chlorine atom,
b) D represents a group -(CH₂)₄-CO-, X denotes a chlorine atom,
c) D represents a group -(CH₂)-₇-CO-, X denotes a chlorine atom,
d) a "Block Copolymer" consisting of units corresponding to the polymers described in paragraphs a) and b);
-4/ the copolymers of acrylic acid and dimethyldiallylammonium chloride.

28. Composition according to Claim 26 or 27, **characterized in that** said cationic or amphoteric polymer(s) is/are present in the proportion of 0.01 to 10% by weight relative to the total weight of the composition.

29. Composition according to any one of the preceding claims, **characterized in that** it comprises at least one surfactant different from the oxyalkylenated or polyglycerolated C₁₂-C₃₀ fatty alcohols chosen from anionic, cationic, nonionic, amphoteric or zwitterionic surfactants in the proportion of at least 0.01% by weight relative to the total weight of the composition.

30. Method for the dyeing of keratinous fibres, in particular human keratinous fibres and more particularly hair, **characterized in that** it consists in applying to the fibres at least one dye composition as defined in any one of Claims 1 to 29, the colour being developed at alkaline, neutral or acidic pH, using an oxidizing composition comprising at least one oxidizing agent which is mixed just at the time of use with the dye composition or which is sequentially applied with no intermediate rinsing.

31. Method according to Claim 30, **characterized in that** the oxidizing agent is chosen from hydrogen peroxide, urea peroxide, alkali metal bromates or ferricyanides, persalts, oxidoreduction enzymes with optionally their respective donor or cofactor.

32. Method according to Claim 31, **characterized in that** the oxidizing agent is a soluton of hydrogen peroxide whose titre varies from 1 to 40 volumes.

33. Method according to any one of Claims 30 to 32, **characterized in that** the pH is between 6 and 11.

34. Method according to any one of Claims 30 to 33, **characterized in that** it consists in applying the composition, freshly prepared at the time of use from the dye and oxidizing compositions, to the dry or wet keratinous fibres, in allowing it to act for a leave-in time varying from 1 to 60 minutes approximately, and preferably from 5 to 45 minutes, in rinsing the fibres, and optionally in washing them with shampoo, and then in rinsing them again, and in drying them.

35. Two-compartment device or kit for dyeing keratinous fibres, in particular human keratinous fibres, and more particularly hair, **characterized in that** the first compartment contains a dye composition as defined in any one of Claims 1 to 29, and the second compartment contains an oxidizing composition.

## Patentansprüche

1. Zusammensetzung zum oxidativen Färben von Keratinfasern, insbesondere menschlichen Keratinfasern und besonders Haaren, die in einem zum Färben geeigneten Medium mindestens einen Oxidationsfarbstoff, mindestens einen C₁₂₋₃₀-Fettalkohol und mindestens einen alkoxylierten oder mehrfach mit Glycerin veretherten C₁₂₋₃₀-Fettalkohol enthält, **dadurch gekennzeichnet, dass** sie ferner 2 bis 15 Gew.-% eines Gemisches aus Glycerin (A) und eines von Glycerin verschiedenen Polyols (B) in einem Gewichtsverhältnis (A) / (B) > 1 enthält.

2. Zusammensetzung nach Anspruch 1, **dadurch gekennzeichnet, dass** das Gemisch aus Glycerin (A) und dem von Glycerin verschiedenen Polyol (B) in Konzentrationen von 5 bis 10 Gew.-%, bezogen auf das Gesamtgewicht der Zusammensetzung, enthalten ist.

3. Zusammensetzung nach Anspruch 1 oder 2, **dadurch gekennzeichnet, dass** das von Glycerin verschiedene Polyol ein Diol ist.

4. Zusammensetzung nach Anspruch 3, **dadurch gekennzeichnet, dass** es sich bei dem Diol um Hexylenglycol handelt.

5. Zusammensetzung nach einem der vorhergehenden Ansprüche, **dadurch gekennzeichnet, dass** das Glycerin in Konzentrationen von 1,5 bis 14 Gew.-%, bezogen auf das Gesamtgewicht der Zusammensetzung, enthalten ist.

6. Zusammensetzung nach einem der vorhergehenden Ansprüche, **dadurch gekennzeichnet, dass** das oder die Polyole, die von Glycerin verschieden sind, in Konzentrationen von 0,5 bis 7 Gew.-%, bezogen auf das Gesamtgewicht der Zusammensetzung, enthalten sind.

7. Zusammensetzung nach einem der vorhergehenden Ansprüche, **dadurch gekennzeichnet, dass** der oder die C₁₂₋₃₀-Fettalkohole in Konzentrationen von 0,1 bis 15 Gew.-%, bezogen auf das Gesamtgewicht der Zusammensetzung, enthalten sind.

8. Zusammensetzung nach Anspruch 7, **dadurch gekennzeichnet, dass** der oder die C₁₂₋₃₀-Fettalkohole in Konzentrationen von 0,5 bis 10 Gew.-%, bezogen auf das Gesamtgewicht der Zusammensetzung, enthalten sind.

9. Zusammensetzung nach einem der vorhergehenden Ansprüche, **dadurch gekennzeichnet, dass** der oder die alkoxylierten oder mehrfach mit Glycerin veretherten C₁₂₋₃₀-Fettalkohole in Konzentrationen von 0,1 bis 15 Gew.-%, bezogen auf das Gesamtgewicht der Zusammensetzung, enthalten sind.

10. Zusammensetzung nach Anspruch 9, **dadurch gekennzeichnet, dass** der oder die alkoxylierten oder mehrfach mit Glycerin veretherten C₁₂₋₃₀-Fettalkohole in Konzentrationen von 0,5 bis 10 Gew.-%, bezogen auf das Gesamtgewicht der Zusammensetzung, enthalten sind.

11. Zusammensetzung nach einem der vorhergehenden Ansprüche, **dadurch gekennzeichnet, dass** der oder die Oxidationsfarbstoffe unter den Oxidationsbasen und/oder Kupplern ausgewählt sind.

12. Zusammensetzung nach einem der vorhergehenden Ansprüche, **dadurch gekennzeichnet, dass** sie mindestens eine Oxidationsbase enthält.

13. Zusammensetzung nach Anspruch 12, **dadurch gekennzeichnet, dass** die Oxidationsbasen und den *o*- oder *p*-Phenylendiaminen, Doppelbasen, *o*- oder *p*-Aminophenolen und heterocyclischen Basen sowie den Additionssalzen dieser Verbindungen mit einer Säure ausgewählt und in Konzentrationen von 0,0005 bis 12 Gew.-%, bezogen auf das Gesamtgewicht der Zusammensetzung, enthalten sind.

14. Zusammensetzung nach Anspruch 11, **dadurch gekennzeichnet, dass** die Kuppler unter *m*-Aminophenolen, *m*-Phenylendiaminen, *m*-Dihydroxybenzolen, Naphtholen, heterocyclischen Kupplern und den Additionssalzen dieser Verbindungen mit einer Säure ausgewählt und in Konzentrationen von 0,0001 bis 15 Gew.-%, bezogen auf das Gesamtgewicht der Zusammensetzung, enthalten sind.

15. Zusammensetzung nach einem der Ansprüche 11 bis 14, **dadurch gekennzeichnet, dass** die Additionssalze der Oxidationsfarbstoffe (Basen und Kuppler) mit einer Säure unter den Hydrochloriden, Hydrobromiden, Sulfaten, Tartraten, Lactaten und Acetaten ausgewählt sind.

16. Zusammensetzung nach einem der vorhergehenden Ansprüche, **dadurch gekennzeichnet, dass** sie ferner mindestens einen Direktfarbstoff in Mengenanteilen von 0,001 bis 20 Gew.-%, bezogen auf das Gesamtgewicht der Zusammensetzung, enthält.

17. Zusammensetzung nach einem der vorhergehenden Ansprüche, **dadurch gekennzeichnet, dass** das zum Färben geeignete Medium ein wässriges Medium oder ein Medium aus Wasser und einem organischen Lösungsmittel ist, das von (A) und (B) verschieden ist.

18. Zusammensetzung nach Anspruch 17, **dadurch gekennzeichnet, dass** die von (A) und (B) verschiedenen organischen Lösungsmittel in Konzentrationen von 0,5 bis 20 Gew.-%, bezogen auf das Gesamtgewicht der Zusammensetzung, enthalten sind.

19. Zusammensetzung nach einem der vorhergehenden Ansprüche, **dadurch gekennzeichnet, dass** sie mindestens ein anionisches, nichtionisches, kationisches oder amphoteres assoziatives Polymer enthält.

20. Zusammensetzung nach Anspruch 19, **dadurch gekennzeichnet, dass** das oder die assoziativen Polymere nichtionisch und unter den Polyetherpolyurethanen ausgewählt sind.

21. Zusammensetzung nach Anspruch 20, **dadurch gekennzeichnet, dass** das Polyetherpolyurethan ein Polykondensat von Polyethylenglycol mit 150 oder 180 mol Ethylenoxid, Stearylalkohol und mindestens einem Diisocyanat ist.

22. Zusammensetzung nach Anspruch 21, **dadurch gekennzeichnet, dass** es sich um Polykondensat von Polyethylenglycol mit 150 oder 180 mol Ethylenoxid, Stearylalkohol und Methylen-bis(4-cyclohexylisocyanat) (SMDI) in einer Menge von 15 Gew.-% in einer Matrix aus Maltodextrin (4 %) und Wasser (81 %) handelt.

23. Zusammensetzung nach Anspruch 20, **dadurch gekennzeichnet, dass** das Polyetherpolyurethan ein Polykondensat von Polyethylenglycol mit 150 oder 180 mol Ethylenoxid, Decylalkohol und mindestens einem Diisocyanat ist.

24. Zusammensetzung nach Anspruch 23, **dadurch gekennzeichnet, dass** das Polyetherpolyurethan ein Polykondensat von Ethylenglycol mit 150 oder 180 mol Ethylenoxid, Decylalkohol und Methylen-bis(4-cyclohexylisocyanat) (SMDI) in einer Menge von 35 Gew.-% in einem Gemisch von Propylenglycol (39 %) und Wasser (26 %) ist.

25. Zusammensetzung nach einem der Ansprüche 19 bis 24, **dadurch gekennzeichnet, dass** das oder die assoziativen Polymere in Mengenanteilen von 0,1 bis 10 Gew.-%, bezogen auf das Gesamtgewicht der Zusammensetzung, enthalten sind.

26. Zusammensetzung nach einem der vorhergehenden Ansprüche, **dadurch gekennzeichnet, dass** sie ferner mindestens ein kationisches oder amphoteres Polymer enthält, das von den in einem der Ansprüche 19 bis 25 beschriebenen assoziativen Polymeren verschieden ist.

27. Zusammensetzung nach Anspruch 26, **dadurch gekennzeichnet, dass** das kationischen oder amphotere Polymer ausgewählt ist unter:
-1 / Homopolymeren von Dimethyldiallylammoniumchlorid;
-2/ Polymeren mit Wiederholungseinheiten der folgenden Formeln (W) oder (U): 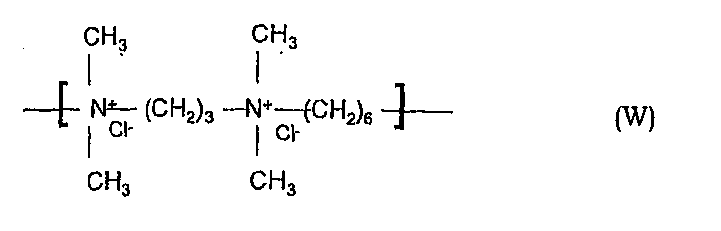 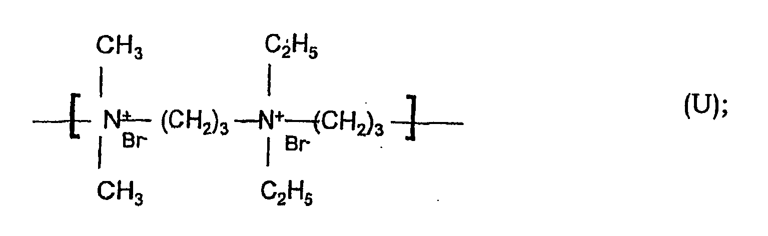
-3/ Polymeren mit Einheiten der folgenden Formel (IX): 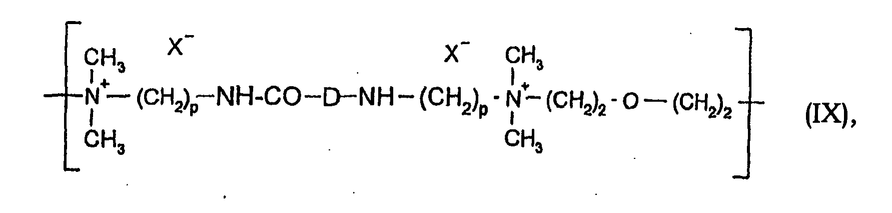 worin p 3 bedeutet und
a) D nicht vorhanden ist und X ein Chloratom bedeutet,
b) D eine Gruppe -(CH₂)₄-CO- ist und X ein Chloratom bedeutet,
c) D eine Gruppe -(CH₂)₇-CO- ist und X ein Chloratom bedeutet und
d) ein "Blockcopolymer", das aus Einheiten gebildet wird, die den unter a) und b) beschriebenen Polymeren entsprechen;
-4/ Copolymeren von Acrylsäure und Dimethyldiallylammoniumchlorid.

28. Zusammensetzung nach Anspruch 26 oder 27, **dadurch gekennzeichnet, dass** das oder die kationischen oder amphoteren Polymere in Mengenanteilen von 0,01 bis 10 Gew.-%, bezogen auf das Gesamtgewicht der Zusammensetzung, enthalten sind.

29. Zusammensetzung nach einem der vorhergehenden Ansprüche, **dadurch gekennzeichnet, dass** sie mindestens einen grenzflächenaktiven Stoff enthält, der von den alkoxylierten oder mehrfach mit Glycerin veretherten C₁₂₋₃₀-Fettalkoholen verschieden ist und unter den anionischen, kationischen, nichtionischen, amphoteren oder zwitterionischen grenzflächenaktiven Stoffen in Mengenanteilen von mindestens 0,01 Gew.-%, bezogen auf das Gesamtgewicht der Zusammensetzung, ausgewählt ist.

30. Verfahren zum Färben von Keratinfasern, insbesondere menschlichen Keratinfasern und besonders Haaren, **dadurch gekennzeichnet, dass** es darin besteht, auf die Fasern mindestens eine Farbmittelzusammensetzung nach einem der Ansprüche 1 bis 29 aufzutragen, wobei die Farbe bei einem alkalischen, neutralen oder sauren pH-Wert mit einer oxidierenden Zusammensetzung gebildet wird, die mindestens ein Oxidationsmittel enthält und die bei der Anwendung mit der Farbmittelzusammensetzung vermischt wird oder ohne zwischenzeitliches Spülen getrennt davon anschließend aufgetragen wird.

31. Verfahren nach Anspruch 30, **dadurch gekennzeichnet, dass** das Oxidationsmittel unter Wasserstoffperoxid, Harnstoffperoxid, Alkalimetallbromaten, Alkalimetallferricyaniden, Persalzen und Redoxenzymen gegebenenfalls mit ihrem jeweiligen Donor oder Cofaktor ausgewählt ist.

32. Verfahren nach Anspruch 31, **dadurch gekennzeichnet, dass** es sich bei dem Oxidationsmittel um eine Wasserstoffperoxidlösung mit einem Titer von 1 bis 40 Volumina handelt.

33. Verfahren nach einem der Ansprüche 30 bis 32, **dadurch gekennzeichnet, dass** der pH-Wert im Bereich von 6 bis 11 liegt.

34. Verfahren nach einem der Ansprüche 30 bis 33, **dadurch gekennzeichnet, dass** es darin besteht, auf die trockenen oder feuchten Keratinfasern die bedarfsgemäß bei der Anwendung aus der Farbmittelzusammensetzung und der oxidierenden Zusammensetzung hergestellte Zusammensetzung aufzutragen, während einer Einwirkzeit von etwa 1 bis 60 min und vorzugsweise 5 bis 45 min einwirken zu lassen, die Fasern zu spülen, gegebenenfalls mit Haarwaschmittel zu waschen, nochmals zu spülen und zu trocknen.

35. Vorrichtung mit 2 Abteilungen oder "Kit" zum Färben von Keratinfasern und insbesondere menschlichen Keratinfasern und besonders Haaren, **dadurch gekennzeichnet, dass** die erste Abteilung eine Farbmittelzusammensetzung nach einem der Ansprüche 1 bis 29 und die zweite Abteilung eine oxidierende Zusammensetzung enthält.
